# EUROPEAN PATENT SPECIFICATION

(11) **EP 4 514 248 B1**
(45) Date of publication and mention of the grant of the patent: **01.07.2026**
(21) Application number: 22724334.2
(22) Date of filing: 29.04.2022
(51) Int. Cl.: A61B 17/34, A61B 17/12, A61B 17/22, A61M 25/00, A61M 25/10

(54) **PERFUSION BALLOON CATHETER**
PERFUSIONSBALLONKATHETER
CATHÉTER À BALLONNET DE PERFUSION

(43) Date of publication of application: 05.03.2025
(73) Proprietor: Bard Peripheral Vascular, Inc., Franklin Lakes, NJ 07417 (US)
(72) Inventor: PALMER, Alex, Tempe, AZ 85281 (US); FORD, Summer, Queen Creek, AZ 85281 (US); SHREVE, Samuel, Scottsdale, AZ 85251 (US)
(74) Representative: advotec.
(86) International application number: PCT/US2022/026905
(87) International publication number: WO 2023/211456

(56) References cited:
- WO-A1-2021/195243
- US-A1- 2007 112 369
- US-A1- 2007 225 750
- US-A1- 2009 112 184
- US-A1- 2018 229 010
- US-B1- 6 196 230
- US-B2- 7 384 412

## Description

### BACKGROUND OF THE INVENTION

Repair of the vasculature often requires an invasive surgical procedure, which is desirable to avoid or at least minimize whenever possible due to the resulting risks and potential complications. Over the years, various endovascular devices, such as balloon catheters, have been developed to perform various types of vascular repairs without such an invasive procedure. This currently includes devices specially adapted for puncturing the vessel wall to allow for the placement of bypass device, such as in a transjugular intrahepatic portosystemic shunt (TIPS) procedure, where a stent graft connects the portal veins to adjacent, lower pressure blood vessels.

Known puncturing devices also typically do not provide a means for isolating the location of the puncture site from blood flow during the procedure. This can add complexity, increase the time required to complete the procedure, and potentially even implicate achieving a successful outcome.

Thus, it would be desirable to provide an apparatus that maintains substantially uninterrupted blood flow in one vessel where such is critical, such as the aorta, while isolating the site of a puncture in an adjacent vessel, such as the renal artery, to facilitate placement of a stent-graft or the like.

Further prior art is known from US 2009/0112184 A1, US 2018/229010 A1, US 2007/225750 A1 and US 6 196 230 B1.

### SUMMARY OF THE INVENTION

An object of the disclosure is to provide an apparatus that maintains substantially uninterrupted blood flow in one vessel where such is critical, such as the aorta, while isolating the site of a puncture in an adjacent vessel, such as the renal artery, to facilitate placement of a stent-graft or the like.

The scope of the invention is set out in the appended set of claims.

According to one aspect of the disclosure, an apparatus for performing a medical procedure in a vessel is provided. The apparatus comprises at least one generally annular inflatable balloon forming an internal passage for allowing perfusion when the at least one generally annular inflatable balloon is inflated. A shaft is connected to the at least one generally annular inflatable balloon, the shaft including an inflation lumen in communication with the at least one generally annular inflatable balloon, and a working lumen including an opening for receiving a retractable needle for puncturing the vessel.

In one embodiment, the inflatable balloon comprises at least two generally annular inflatable balloons interconnected by a membrane. In another embodiment, the inflatable balloon comprises at least two pairs of generally annular inflatable balloons, each pair of balloons interconnected by a membrane. In any case, the membrane is fluid-impervious, and may be tubular in shape.

In one embodiment, the inflatable balloon comprises a first inflatable generally annular balloon having a first inflated diameter that is smaller than a second inflated diameter of a second generally annular inflatable balloon. The apparatus may further include third and fourth inflatable balloons having a generally annular shape, the third inflatable balloon having a third inflated diameter that is smaller than a fourth inflated diameter of the fourth annular inflatable balloon. A distal end of the shaft passes at least partially through a wall of the annular inflatable balloon to allow a needle to pass therethrough. In one version, the inflatable balloon is elongated in an axial direction.

According to another aspect of the disclosure, an apparatus for forming a catheter for use in performing a medical procedure in a vessel is provided. The apparatus comprises first and second inflatable balloons, each of the first and second inflatable balloons having a generally annular shape. A first membrane connects the first and second inflatable balloons. The first membrane may be fluid-impervious, and may be tubular in shape.

In one embodiment, the first inflatable balloon has a first inflated diameter that is smaller than a second inflated diameter of the second inflatable balloon. The apparatus may further include third and fourth inflatable balloons having a generally annular shape, and a second membrane connecting the third and fourth inflatable balloons. The third inflatable balloon has a third inflated diameter smaller than a fourth inflated diameter of the fourth inflatable balloon.

The apparatus may also include a shaft having an inflation lumen. The shaft may also have a working lumen for receiving a needle adapted for exiting the shaft to puncture the vessel, such as in a space between two inflatable balloons (or two pairs of inflatable balloons). The shaft may also include a guidewire lumen.

A further aspect of this disclosure pertains to an apparatus for performing a medical procedure in a vessel. The apparatus comprises first and second inflatable balloons, and a shaft for supporting the first and second inflatable balloons. The shaft includes a working lumen for receiving a retractable needle adapted for exiting the shaft in a space between the balloons to puncture the vessel.

In one embodiment, a first membrane connects the first and second inflatable balloons. The first membrane is fluid-impervious and may be tubular.

The first inflatable balloon has a first inflated diameter that is smaller than a second inflated diameter of the second inflatable balloon. The apparatus may further include third and fourth inflatable balloons having a generally annular shape, and a second membrane connecting the third and fourth inflatable balloons. The third inflatable balloon has a third inflated diameter that is smaller than a fourth inflated diameter of the fourth inflatable balloon. The shaft may further include a guidewire lumen and an inflation lumen, and the first and second inflatable balloons are generally annular in shape, as may be the third and fourth inflatable balloons.

The method also pertains to an apparatus for performing a medical procedure in a main vessel connected to a branch vessel. The apparatus comprises a first inflatable balloon forming an internal passage for allowing perfusion in the main vessel when the first inflatable balloon is inflated. A shaft is connected to the first inflatable balloon, the shaft including an inflation lumen in communication with the first inflatable balloon. A second inflatable balloon is connected to the shaft for insertion into the branch vessel when inflated, the second balloon having an annular shape.

In one embodiment, a first membrane connects the first and second inflatable balloons. The first membrane may be fluid-impervious, and may be tubular.

The first inflatable balloon may have a first inflated diameter that is smaller than a second inflated diameter of the second inflatable balloon. Third and fourth inflatable balloons may also be provided having a generally annular shape, and a second membrane connecting the third and fourth inflatable balloons. The first inflatable balloon may have a first inflated diameter that is smaller than a second inflated diameter of the second inflatable balloon. The third inflatable balloon may have a third inflated diameter that is smaller than a fourth inflated diameter of the fourth inflatable balloon. The shaft may have a guidewire lumen and an inflation lumen, and the first inflatable balloon may be annular.

Yet a further aspect of the disclosure relates to a method of performing a medical procedure at a junction between first and second vessels having a first fluid flow and a second, transverse fluid flow. The method comprises inflating a balloon catheter at the junction so as to allow the first fluid flow while obstructing the second fluid flow. The method may further include the step of puncturing one of the first or second vessels, which may comprise deploying a needle from the balloon catheter.

### BRIEF DESCRIPTION OF THE DRAWINGS

A better understanding of the above and further advantages of the present disclosure results from referring to the following description in conjunction with the accompanying drawings in which:
Figure 1 is a schematic view of a first embodiment of a perfusion balloon catheter;
Figure 1A is a cross-sectional view taken along line 1A-1A of Figure 1;
Figure 2 is a schematic view of a second embodiment of a perfusion balloon catheter;
Figure 3 is a side view of a third embodiment of a perfusion balloon catheter; and
Figures 3A, 3B, 3C, and 3D illustrate various portions of the catheter of Figure 3.

The dimensions of some of the elements may be exaggerated relative to other elements for clarity or several physical components may be included in one functional block or element. Further, sometimes reference numerals may be repeated among the drawings to indicate corresponding or analogous elements. Moreover, some of the parts depicted in the drawings may be combined into a single function.

### DETAILED DESCRIPTION

In the following detailed description, numerous specific details are set forth to provide a thorough understanding of the presently disclosed invention(s). The disclosed embodiments may be practiced without these specific details. In other instances, well-known methods, procedures, components, or structures may not have been described in detail so as not to obscure the disclosed inventive concepts.

The invention of this disclosure is not limited in its application to the details of construction and the arrangement of the components set forth in the following description or illustrated in the drawings. The inventive concepts disclosed are capable of other embodiments or of being practiced or carried out in various ways. It is also to be understood that the phraseology and terminology employed herein are for the purpose of description and should not be regarded as limiting. The invention is defined by the claims.

Certain features of the disclosed embodiments that are, for clarity, described in the context of separate embodiments, may also be provided in combination in a single embodiment. Conversely, various features of the invention that are, for brevity, described in the context of a single embodiment, may also be provided separately or in any suitable sub-combination.

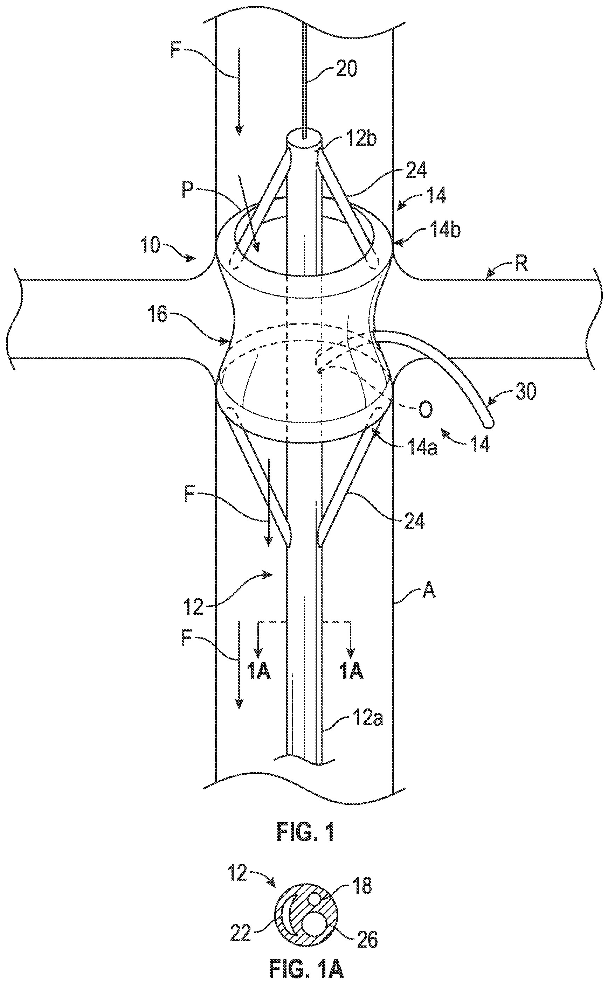

With reference to Figure 1, a perfusion catheter 10 is provided for endovascular use and, in particular, one that may be adapted for use in selectively puncturing a vessel, such as for forming a shunt using a stent-graft or the like. The catheter 10 includes a shaft 12 having a proximal end 12a and a distal end 12b. The distal end 12b of the shaft 12 supports an expandable element, which in the illustrated embodiment comprises of a pair of spaced, inflatable balloons 14.

The pair of balloons 14 are spaced apart in an axial direction, and interconnected indirectly by the shaft 12, as well as directly by a connector, such as a flexible membrane 16 spanning between the balloons. In one example, this membrane 16 is tubular and formed of a polymer material, such as polytetrafluoroethylene, and thus be substantially fluid-impervious. The balloons 14 are annular in shape in the inflated condition, as shown in Figure 1, such that the shaft 12 passes through each along an internal passage P, and the membrane 16 is peripherally attached, such as by adhesives, welding, or the like. The balloons 14 may be complaint or semi-compliant, meaning they may conform to the shape of an external structure when inflated, such as the inner wall of a vessel. When deflated, the balloons 14 fold or collapse onto the shaft 12, along with the connected tubular membrane 16 (which is the position prior to insertion into the vessel).

As can be understood from Figure 1A, the shaft 12 includes a plurality of internal passages extending from the proximal end 12a to the distal end 12b. These passages include a guidewire lumen 18 for receiving a guidewire 20 for guiding the catheter 10 to a position in the vasculature, which extends fully through the shaft 12. The shaft 12 also includes an inflation lumen 22, which may communicate with the balloons 14 via a plurality of necks 24 connecting the balloons to the shaft 12 at proximal and distal points along the distal end 12b thereof.

A working lumen 26 may also be provided in the shaft 12 for receiving an elongated puncture needle 30. When advanced a suitable distance, such as by pushing a proximal end, a distal end of this needle 30 may alight through an opening O (shown in phantom) formed along a distal end 12b of the shaft 12, such as between the balloons 14, to puncture a vessel wall, and then retract into the working lumen. The location of the opening O in the shaft 12 may include an identifier to allow for visualization under fluoroscopy, such as for example a radiopaque marker. A suitable hub (not shown) may also be provided at the proximal end 12a of the shaft 12 for communicating with each lumen 18, 22, 26.

In use, the catheter 10 may be inserted into a main vessel, such as the aorta A, from a remote location (such as the iliac artery) via guidance provided by a previously installed guidewire 20. Using fluoroscopy (to identify the location of one or more radiopaque markings on the shaft 12, as noted below), the catheter 10 may be advanced to a location of a branch vessel, such as the renal artery R, where a shunt is desired. The positioning is such that the distal balloon 14b is located distally of the branch vessel, and the proximal balloon 14a is located proximally of the branch vessel, as shown in Figure 1.

The balloons 14 may then be inflated via inflation lumen 22 and necks 24 from a remote inflation source, such as an indeflator connected to a hub at a portion of the proximal end 12a of the shaft 12 external to the patient. Consequently, the annular balloons 14 expand and engage the inner wall of the main vessel (e.g., aorta A), as illustrated. Despite this expansion, the open annular nature of the balloons 14 allows blood flow F to perfuse, including through a passage P extending axially through the membrane 16 in view of its natural expansion upon inflation of the balloons.

As can be appreciated, when balloons 14 are inflated, transverse or cross fluid flow through the branch vessel, such as the renal artery R, is substantially occluded by the presence of the expanded membrane 16. The needle 30 may then be advanced through the working lumen 26, until the tip thereof extends out through the corresponding opening O in the shaft 12.

The needle 30 may be passed through the membrane 16 (a passage or opening, such as a hermetically sealed port in the membrane wall, may be provided for this purpose, as noted below), and into engagement with the branch vessel, such as renal artery R, to puncture the same. This may be facilitated by forming the needle of a shape memory material, such as Nitinol, which may induces a curvature to allow for the needle 30 to enter and puncture the branch vessel in a preferential manner, as illustrated. As can be appreciated, extravasation is prevented at the puncture site by the presence of the balloon 14 and membrane 16 in particular.

Once the puncture is completed, the needle 30 is retracted into the shaft 12 and the operation completed (such as by inserting a bypass graft, not shown). The balloons 14 may be deflated and refolded (such as by using a sheath, not shown). The catheter 10 may then be withdrawn from the vessel via the guidewire 20, or positioned at another location for further use in performing a procedure.

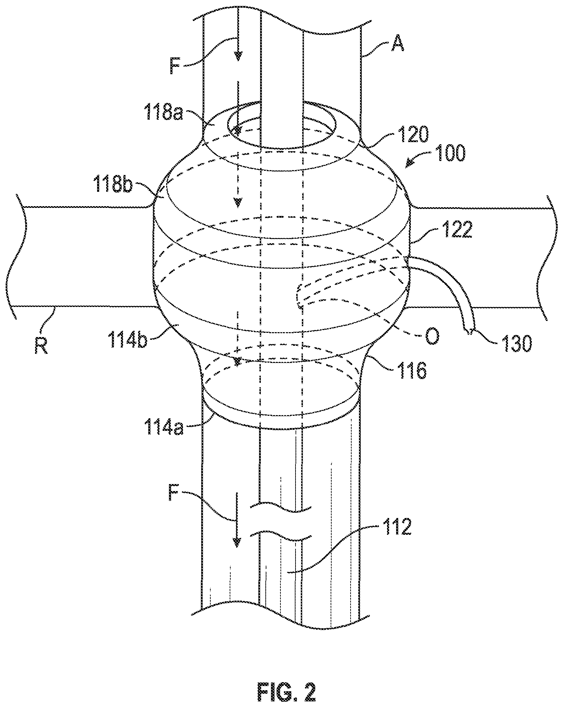

Turning to Figure 2, an alternative embodiment is shown. In this version of catheter 100, four balloons are provided, including two proximal annular balloons 114a, 114b connected by a first membrane 116, and two distal annular balloons 118a, 118b connected by a second membrane 120. The innermost balloons 114b, 118b may also be connected by a membrane 122, and may be larger in diameter when inflated than the outermost balloons 114a, 118a. The balloons 114a, 114b, 118a, 118b together with membranes 116, 120, 122 may be supported by a distal end 112a of the shaft 112, which may have a similar cross-section to shaft 12, and thus may be inflated or deflated in a similar manner.

When located at a branched location in the vasculature, such as for example the meeting of the aorta A and the renal artery R, the balloons 114a, 114b, 118a, 118b may be inflated, such as via independent inflation necks in fluid communication with the shaft (see, e.g., Figure 1). In view of the different diameters provided, the smaller, outermost balloons 114a, 118a engage along the inner wall of the main vessel (e.g., the aorta A), whereas the inner balloons 114b, 118b may extend partially into the branch vessel (e.g., the renal artery R). This dual engagement provides a stabilizing function and prevents slippage, facilitating the advance of a needle 130 as desired for puncturing, which may be in the manner previously described (e.g., through an opening O in the shaft 12, through a passage or opening in the membrane 122, and into engagement with the vessel wall at the desired location, including possibly with curved shape attainment of the needle 130 via use of a memory material), as well as the insertion of a bypass graft. While axial flow F continues in view of the generally annular shape, cross or transverse flow is also occluded by the presence of the membranes 116, 120, 122, and extravasation is again prevented at the puncture site. Once the needle 130 is withdrawn and the operation completed, the balloons 114a, 114b, 118a, 118b may be deflated, and the catheter 100 withdrawn from the vessel via a guidewire (not shown) via a lumen in shaft 112 or relocated for further use, as desired.

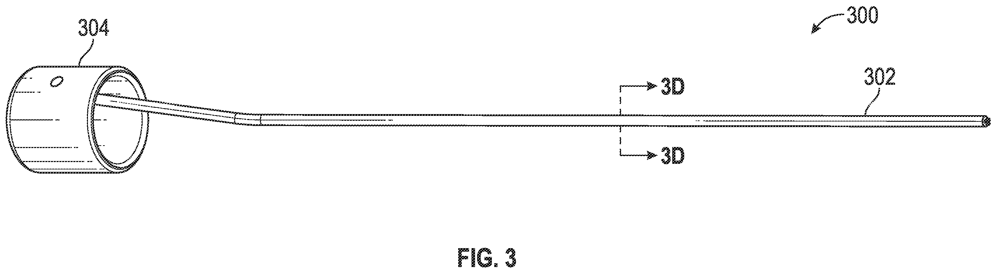

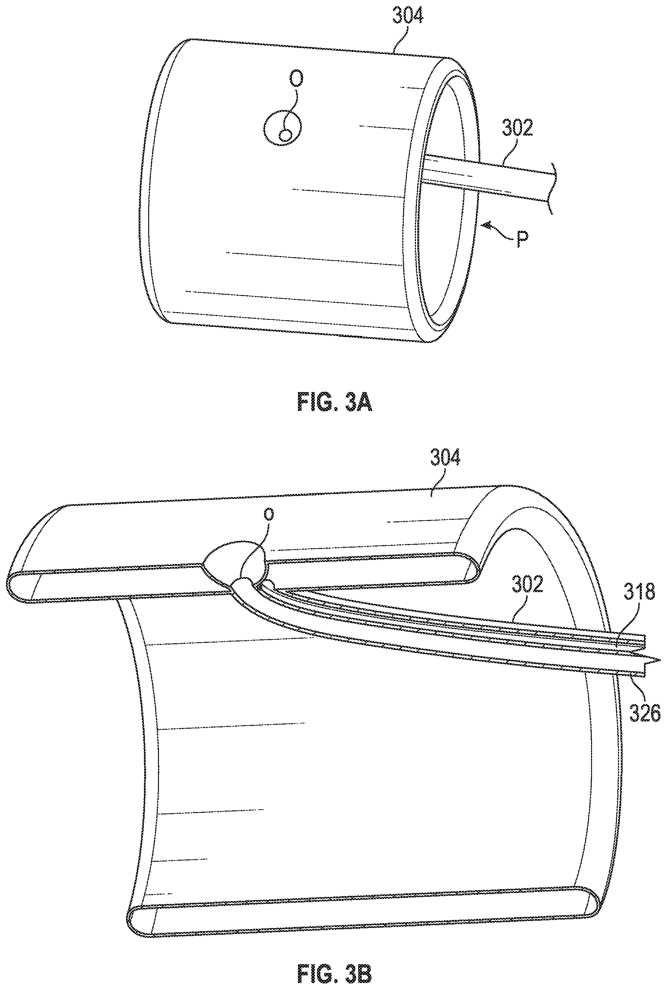

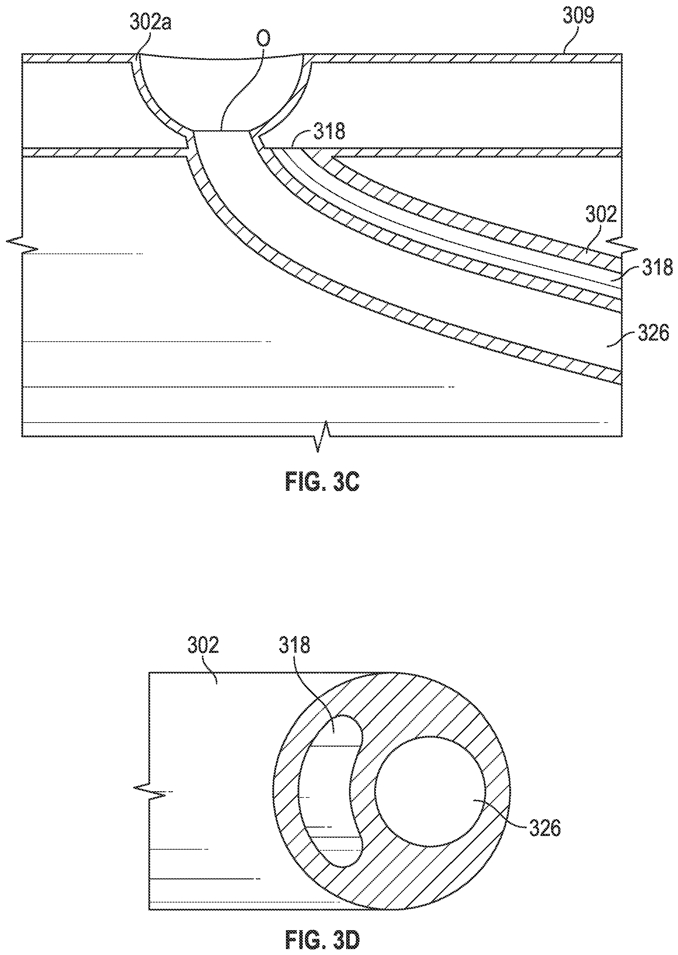

Referring now to Figures 3 and 3A-3D, a further embodiment of a catheter 300 includes a shaft 302 connected with a perfusion balloon 304 at a distal end. With further reference to Figures 3A and 3B, it can be understood that the balloon 302 comprises an annular structure elongated in an axial direction, and thus forming a tube when inflated with an internal passage P to allow for perfusive blood flow. Inflation and deflation may be achieved by an inflation lumen 318 in the shaft 302 (see Figure 3D), which may communicate at a distal end with the interior of the balloon 304 in view of a connection with a distal end 302a of the shaft 302 (see Figures 3B and 3C), which passes through the balloon.

A working lumen 326 in the shaft 302 also extends to the distal end 302 and through an opening O, which may be recessed in an outer surface of the balloon 304 within an oversized, cup-shaped or hemispherical portion of the distal tip of shaft. The working lumen 302 may be curved along a distal end, as shown, to guide the needle to a transverse orientation relative to an adjacent vessel or vessel wall. A guidewire lumen (not shown) may also be provided in shaft 302 for guiding the catheter 300 to a treatment site.

Thus, when positioned in a vessel and inflated, the balloon 304 allows for blood to remain flowing through a central passage in view of its annular shape. In the inflated condition, a puncture needle (not shown) or the like may be passed through working lumen 326 and guided into engagement with an adjacent vessel wall, such as for puncturing the same (or an adjacent branch vessel) for installation of a stent graft or the like. Extravasation is again prevented by the surrounding wall of the balloon 304 in contact with the adjacent vessel wall. Once the needle is withdrawn and the operation completed the balloons 304 may be deflated, and the catheter 300 withdrawn from the vessel.

As used herein, the following terms have the following meanings:
"A", "an", and "the" as used herein refers to both singular and plural referents unless the context clearly dictates otherwise. By way of example, "a compartment" refers to one or more than one compartment.

"About," "substantially," "approximately," or "generally" as used herein referring to a measurable value, such as a parameter, an amount, a temporal duration, and the like, is meant to encompass variations of +/- 20% or less, preferably +/-10% or less, more preferably +/-5% or less, even more preferably +/-1 % or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows, e.g., component or the like does not exclude or preclude the presence of additional, non-recited components, features, element, members, steps, known in the art or disclosed therein.

Although the invention is described in conjunction with specific embodiments, many alternatives, modifications, and variations will be apparent to those skilled in the art. Accordingly, this disclosure embraces all such alternatives, modifications, and variations that fall within the scope of the appended claims. It is to be fully understood that certain aspects, characteristics, and features, of the invention, which are, for clarity, illustratively described and presented in the context or format of a plurality of separate embodiments, may also be illustratively described and presented in any suitable combination or sub-combination in the context or format of a single embodiment. Conversely, various aspects, characteristics, and features, of the invention which are illustratively described and presented in combination or sub-combination in the context or format of a single embodiment may also be illustratively described and presented in the context or format of a plurality of separate embodiments.

The invention is defined by the following claims.

## Claims

1. An apparatus for performing a medical procedure in a vessel, comprising:
at least one generally annular inflatable balloon (14, 14a, 14b, 114a, 114b, 118a, 118b, 302, 304) forming an internal passage for allowing perfusion when the at least one annular inflatable balloon (14, 14a, 14b, 114a, 114b, 118a, 118b, 302, 304) is inflated; and
a shaft (12, 112, 302) connected to the at least one generally annular inflatable balloon (14, 14a, 14b, 114a, 114b, 118a, 118b, 302, 304), the shaft (12, 112, 302) including an inflation lumen (22, 318) in communication with the at least one generally annular inflatable balloon (14, 14a, 14b, 114a, 114b, 118a, 118b, 302, 304), and a working lumen (26) including an opening (O) for passing a tool from the working lumen (26) into the vessel;
**characterised in that** the at least one annular inflatable balloon (14, 14a, 14b, 114a, 114b, 118a, 118b, 302, 304) comprises (i) at least two generally annular inflatable balloons (14, 14a, 14b, 302, 304) interconnected by a membrane (16), and wherein the opening (O) is disposed between the at least two generally annular inflatable balloons (14, 14a, 14b, 302, 304); or (ii) at least two pairs of generally annular inflatable balloons (114a, 114b, 118a, 118b), each pair of generally annular balloons (114a, 114b, 118a, 118b) interconnected by a membrane (116, 120, 122), and wherein the opening (O) is disposed between the at least two pairs of generally annular inflatable balloons (114a, 114b, 118a, 118b).

2. The apparatus of claim 1, wherein the membrane (16, 116, 120, 122) is fluid-impervious.

3. The apparatus of claim 1 or 2, wherein the membrane (16, 116, 120, 122) is tubular.

4. The apparatus of claim 1, wherein the at least one generally annular inflatable balloon (114a, 114b, 118a, 118b) comprises a first inflatable generally annular balloon (114a, 118a) having a first inflated diameter that is smaller than a second inflated diameter of a second annular generally inflatable balloon (114b, 118b).

5. The apparatus of claim 4, further including third and fourth generally annular inflatable balloons having a generally annular shape, the third annular generally annular inflatable balloon having a third inflated diameter that is smaller than a fourth inflated diameter of the fourth annular inflatable balloon, wherein the second and fourth inflatable balloons (114b, 118b) are positioned in between the first and third generally annular inflatable balloons (114a, 118a) and are connected to the membrane.

6. The apparatus of claim 1, wherein a distal end of the shaft (12) passes at least partially through a wall of the at least one generally annular inflatable balloon (14, 14a, 14b, 114a, 114b, 118a, 118b, 302, 304) to allow a needle (30, 130) to pass therethrough.

7. The apparatus of claim 1, wherein the at least one generally annular inflatable balloon (14, 14a, 14b, 114a, 114b, 118a, 118b, 302, 304) is elongated in an axial direction.

## Patentansprüche

1. Vorrichtung zur Durchführung eines medizinischen Eingriffs in einem Gefäß, Folgendes umfassend:
mindestens einen im Allgemeinen ringförmigen aufblasbaren Ballon (14, 14a, 14b, 114a, 114b, 118a, 118b, 302, 304), der einen Innendurchgang ausbildet, um eine Durchblutung zu ermöglichen, wenn der mindestens eine ringförmige aufblasbare Ballon (14, 14a, 14b, 114a, 114b, 118a, 118b, 302, 304) aufgeblasen ist; und
einen Schaft (12, 112, 302), der mit dem mindestens einen allgemein ringförmigen aufblasbaren Ballon (14, 14a, 14b, 114a, 114b, 118a, 118b, 302, 304) verbunden ist, wobei der Schaft (12, 112, 302) ein mit dem mindestens einen im Allgemeinen ringförmigen aufblasbaren Ballon (14, 14a, 14b, 114a, 114b, 118a, 118b, 302, 304) in Verbindung stehendes Aufblaslumen (22, 318) und ein eine Öffnung (O) zum Führen eines Instruments aus dem Arbeitslumen (26) in das Gefäß umfassendes Arbeitslumen (26) umfasst;
**dadurch gekennzeichnet, dass**
der mindestens eine ringförmige aufblasbare Ballon (14, 14a, 14b, 114a, 114b, 118a, 118b, 302, 304) Folgendes umfasst: (i) mindestens zwei durch eine Membran (16) miteinander verbundene im Allgemeinen ringförmige aufblasbare Ballons (14, 14a, 14b, 302, 304), und wobei die Öffnung (O) zwischen den mindestens zwei im Allgemeinen ringförmigen aufblasbaren Ballons (14, 14a, 14b, 302, 304) angeordnet ist; oder (ii) mindestens zwei Paare von im Allgemeinen ringförmigen aufblasbaren Ballons (114a, 114b, 118a, 118b), wobei immer zwei im Allgemeinen ringförmige Ballons (114a, 114b, 118a, 118b) jeweils durch eine Membran (116, 120, 122) miteinander verbunden sind, und wobei die Öffnung (O) zwischen den mindestens zwei Paaren von im Allgemeinen ringförmigen aufblasbaren Ballons (114a, 114b, 118a, 118b) angeordnet ist.

2. Vorrichtung nach Anspruch 1, wobei die Membran (16, 116, 120, 122) fluidundurchlässig ist.

3. Vorrichtung nach Anspruch 1 oder 2, wobei die Membran (16, 116, 120, 122) rohrförmig ist.

4. Vorrichtung nach Anspruch 1, wobei der mindestens eine im Allgemeinen ringförmige aufblasbare Ballon (114a, 114b, 118a, 118b) einen ersten aufblasbaren im Allgemeinen ringförmigen Ballon (114a, 118a) mit einem ersten Durchmesser im aufgeblasenen Zustand umfasst, der kleiner als ein zweiter Durchmesser im aufgeblasenen Zustand eines zweiten im Allgemeinen ringförmigen aufblasbaren Ballons (114b, 118b) ist.

5. Vorrichtung nach Anspruch 4, des Weiteren umfassend einen dritten und einen vierten im Allgemeinen ringförmigen aufblasbaren Ballon mit einer im Allgemeinen ringförmigen Form, wobei der dritte ringförmige im Allgemeinen ringförmige aufblasbare Ballon einen dritten Durchmesser im aufgeblasenen Zustand aufweist, der kleiner als ein vierter Durchmesser im aufgeblasenen Zustand des vierten ringförmigen aufgeblasenen Ballons ist, wobei der zweite und der vierte aufblasbare Ballon (114b, 118b) zwischen dem ersten und dem dritten im Allgemeinen ringförmigen aufblasbaren Ballon (114a, 118a) positioniert sind und mit der Membran verbunden sind.

6. Vorrichtung nach Anspruch 1, wobei ein distales Ende des Schafts (12) zumindest teilweise durch eine Wand des mindestens einen im Allgemeinen ringförmigen aufblasbaren Ballons (14, 14a, 14b, 114a, 114b, 118a, 118b, 302, 304) durchtritt, um zu ermöglichen, dass eine Nadel (30, 130) dort hindurchgehen kann.

7. Die Vorrichtung nach Anspruch 1, wobei der mindestens eine im Allgemeinen ringförmige aufblasbare Ballon (14, 14a, 14b, 114a, 114b, 118a, 118b, 302, 304) in einer axialen Richtung lang gestreckt ist.

## Revendications

1. Appareil pour effectuer un acte médical dans un vaisseau, ledit appareil comprenant :
au moins un ballonnet (14, 14a, 14b, 114a, 114b, 118a, 118b, 302, 304) gonflable et généralement annulaire formant un passage interne pour permettre une perfusion lorsque l'au moins un ballonnet (14, 14a, 14b, 114a, 114b, 118a, 118b, 302, 304) gonflable et annulaire est gonflé ; et
une tige (12, 112, 302) reliée à l'au moins un ballonnet (14, 14a, 14b, 114a, 114b, 118a, 118b, 302, 304) gonflable et généralement annulaire, la tige (12, 112, 302) comprenant une lumière de gonflage (22, 318) en communication avec l'au moins un ballonnet (14, 14a, 14b, 114a, 114b, 118a, 118b, 302, 304) gonflable et généralement annulaire et une lumière de travail (26) comprenant une ouverture (O) pour passer un instrument de la lumière de travail (26) dans le vaisseau ;
**caractérisé en ce que**
l'au moins un ballonnet (14, 14a, 14b, 114a, 114b, 118a, 118b, 302, 304) gonflable et annulaire comprend (i) au moins deux ballonnets (14, 14a, 14b, 302, 304) gonflables et généralement annulaires qui sont reliés l'un à l'autre par une membrane (16), et dans lequel l'ouverture (O) est disposée entre les au moins deux ballonnets (14, 14a, 14b, 302, 304) gonflables et généralement annulaires, ou (ii) au moins deux paires de ballonnets (114a, 114b, 118a, 118b) gonflables et généralement annulaires, toujours deux ballonnets (114a, 114b, 118a, 118b) généralement annulaires étant reliés par une membrane (116, 120, 122) à chaque fois, et dans lequel l'ouverture (O) est disposée entre les au moins deux paires de ballonnets (114a, 114b, 118a, 118b) gonflables et généralement annulaires.

2. Appareil selon la revendication 1, dans lequel la membrane (16, 116, 120, 122) est imperméable à des fluides.

3. Appareil selon la revendication 1 ou 2, dans lequel la membrane (16, 116, 120, 122) est tubulaire.

4. Appareil selon la revendication 1, dans lequel l'au moins un ballonnet (114a, 114b, 118a, 118b) gonflable et généralement annulaire comprend un premier ballonnet (114a, 118a) généralement annulaire et gonflable ayant un premier diamètre à l'état gonflé qui est inférieur à un deuxième diamètre à l'état gonflé d'un deuxième ballonnet (114b, 118b) généralement gonflable et annulaire.

5. Appareil selon la revendication 4, comprenant en outre un troisième et un quatrième ballonnet gonflable et généralement annulaire ayant une forme généralement annulaire, le troisième ballonnet gonflable et généralement annulaire et annulaire ayant un troisième diamètre à l'état gonflé qui est inférieur à un quatrième diamètre à l'état gonflé d'un quatrième ballonnet gonflable et annulaire, dans lequel le deuxième et le quatrième ballonnet (114b, 118b) gonflables sont positionnés entre le premier et le troisième ballonnet (114a, 118a) gonflables et généralement annulaires et sont reliés à la membrane.

6. Appareil selon la revendication 1, dans lequel une extrémité distale de la tige (12) passe au moins partiellement à travers une paroi de l'au moins un ballonnet (14, 14a, 14b, 114a, 114b, 118a, 118b, 302, 304) gonflable et généralement annulaire pour permettre qu'une aiguille (30, 130) puisse passer à travers celle-ci.

7. Appareil selon la revendication 1, dans lequel l'au moins un ballonnet (14, 14a, 14b, 114a, 114b, 118a, 118b, 302, 304) gonflable et généralement annulaire est allongé dans une direction axiale.
